(19) Europäisches Patentamt
European Patent Office
Office européen des brevets

(11) EP 1 397 200 B1

(12) **EUROPÄISCHE PATENTSCHRIFT**

(45) Veröffentlichungstag und Bekanntmachung des Hinweises auf die Patenterteilung:
**25.04.2007 Patentblatt 2007/17**

(21) Anmeldenummer: **02740675.0**

(22) Anmeldetag: **31.05.2002**

(51) Int Cl.:
***B01J 13/02*** *(2006.01)*

(86) Internationale Anmeldenummer:
**PCT/EP2002/006001**

(87) Internationale Veröffentlichungsnummer:
**WO 2002/096551 (05.12.2002 Gazette 2002/49)**

(54) **AUFLÖSBARE NANO- UND MIKROKAPSELN, VERFAHREN ZU IHRER HERSTELLUNG SOWIE IHRE VERWENDUNG**

DISSOLVABLE NANOCAPSULES AND MICROCAPSULES, METHOD FOR THE PRODUCTION THEREOF AND THEIR USE

MICROCAPSULES ET NANOCAPSULES SOLUBLES, PROCEDE DE PRODUCTION ASSOCIE ET UTILISATION

(84) Benannte Vertragsstaaten:
**AT BE CH CY DE DK ES FI FR GB GR IE IT LI LU MC NL PT SE TR**

(30) Priorität: **31.05.2001 DE 10127526**

(43) Veröffentlichungstag der Anmeldung:
**17.03.2004 Patentblatt 2004/12**

(73) Patentinhaber: **novosom AG**
**06120 Halle (DE)**

(72) Erfinder:
- **Dr. PANZNER, Steffen**
  **06114 Halle (DE)**
- **Dr. ESSLER, Frank**
  **55131 Mainz (DE)**

(74) Vertreter: **Ziebig, Marlene**
**Wallstrasse 58/59**
**10179 Berlin (DE)**

(56) Entgegenhaltungen:
**EP-A- 0 681 834** **WO-A-02/09864**
**WO-A-02/31092** **DE-A- 19 810 965**

- **WEN S ET AL: "Microcapsules through polymer complexation - Part 3: encapsulation and culture of human Burkitt lymphoma cells in vitro" BIOMATERIALS, ELSEVIER SCIENCE PUBLISHERS BV., BARKING, GB, Bd. 16, Nr. 4, 1995, Seiten 325-335, XP004033057 ISSN: 0142-9612**
- **MEYENBURG S ET AL: "Fibrin encapsulated liposomes as protein delivery system - Studies on the in vitro release behavior" JOURNAL OF CONTROLLED RELEASE, ELSEVIER SCIENCE PUBLISHERS B.V. AMSTERDAM, NL, Bd. 69, Nr. 1, 3. Oktober 2000 (2000-10-03), Seiten 159-168, XP004217542 ISSN: 0168-3659**

EP 1 397 200 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft auflösbare Nano- und Mikrokapseln, Verfahren zu ihrer Herstellung sowie die Verwendung der Nano- und Mikrokapseln zur Verpackung und Freisetzung von wirkstoffen.

Bekannte Nanokapseln aus Polymeren können nach verschiedenen Verfahren hergestellt werden, wobei die Herstellung der Nanokapseln ihre Charakteristik - wie Stabilität, oder Auflöseverhalten - mit bestimmt. So sind beispielsweise mehrere templatbasierte Herstellungsmethoden bekannt, bei denen bereits vorhandene Partikel wie Latizes, Liposomen oder Emulsionen mit einer in sich stabilen Hüllschicht überzogen werden. Solche Partikel werden in der DE 198 12 083, WO 00/28972, WO 01/64330, DE 100 01 172 oder WO 00/03797 offenbart.

Die beschriebenen Strukturen zeichnen sich durch einfache Herstellbarkeit, Lagerstabilität und physiologische Anwendbarkeit aus, woraus ihr Einsatz bei biochemischen und pharmazeutischen Anwendungen resultiert. Für eine Reihe von Anwendungen, insbesondere im Bereich des Advanced Drug Delivery, gilt das Konzept, das die aufgebrachten Hüllschichten das Templat schützen oder dessen Oberfläche verändern, etwa um eine verlängerte biologische Halbwertszeit zu erreichen. Wirkstoffe können sich dabei im Innern der Struktur befinden oder Bestandteile der Hüllschichten sein.

Die Stabilität der Hüllschichten ist wegen der Vielzahl der vorhandenen Bindungen extrem hoch. So offenbart die DE 198 12 083 einen Erhalt der Hüllschichten auch nach Calcinierung des Templats bei 500˚C oder nach Auflösung des Templats durch starke Säuren, in der WO 00/03797 ist die Stabilität der Strukturen unter verschiedenen pH-Bedingungen und Ionenstärken gezeigt.

So wünschenswert die hohe Stabilität der Hüllstrukturen für eine Lagerung und für den Transport der Wirkstoffe ist, so nachteilig ist diese Stabilität, wenn die Wirkstoffe am Wirkort schnell und effektiv freigesetzt werden sollen. Derzeit sind keine stabilen Nanokapseln - oder Verfahren zu ihrer Herstellung - bekannt, die sich trotz ihrer Stabilität einfach und schnell auflösen. Dies ist nachteilig, da die für verschiedene klinische oder forschungsrelevante Anwendungen zahlreiche stabile Nanokapslen benötigt werden, die sich unter definierten Bedingungen gut und schnell auflösen.

Aufgabe der Erfindung war es daher, leicht auflösbare stabile Nanokapseln bereitzustellen.

Die Erfindung löst dieses technische Problem durch die Bereitstellung von auflösbaren Nano- oder Mikrokapseln, umfassend ein kolloidales Templat in wässriger Lösung und komplementär geladene Polymere auf dem Templat, wobei mindestens eines der Polymere ein Polyampholyt ist, welcher durch eine pH-Wert Änderung ent- oder umladbar ist.

Es wurde überraschend gefunden, dass die erfindungsgemäßen Nanokapseln, stabil sind und sich durch geringe Änderungen des pH-Milieus auflösen lassen. Sie sind daher besonders gut geeignet, Wirkstoffe zu transportieren und selektiv wieder freizusetzen. Es wurde also gefunden, dass trotz des sehr stabilen Aufbaus der Kapseln immer noch eine Umladung der Komponenten stattfinden kann und die Kapseln danach zerfallen.

Unter Polyelektrolyten versteht man im Sinne der Erfindung wasserlösliche Verbindungen, die unter den Reaktionsbedingungen, insbesondere bei dem benutzten pH-Wert eine Vielzahl ionisch geladener Gruppen besitzen. Je nach Art der dissoziierbaren Gruppe kann man diese Polylelektrolyte sicher als Polysäuren oder Polybasen klassifizieren. Polysäuren sind etwa Polyvinylschwefelsäure, Polystyrensulfonsäure, Polyacrylsäure, Alginsäure, Pektin, Heparin, Dextransulfat, Nukleinsäuren, Polyglutaminsäure, Polymaleinsäure und andere mehr, etwa Copolymere aus ungeladenen und säuretragenden Monomeren. Polybasen sind beispielsweise Chitosan, Polyethylenimin, Polyallylamin, Polylysin, Polyarginin, Polyquaternium und andere mehr. Weitere Polyelektrolyte sind dem Fachmann z.B. aus der WO 00/28972 oder WO 00/03797 bekannt, die in den Offenbarungsgehalt der Erfindung mit aufgenommen sind. Diese Schriften beschreiben auch einige geeignete Methoden zur Abscheidung der Polyelektrolyte.

Die Nanokapseln können beispielsweise so hergestellt werden, dass ein oder mehrere Polyampholyte Bestandteil der Hüllschicht sind. Dazu werden die Polyampholyte in einem ihrer zwei definierten Ladungszustände, als Polyanion oder Polykation, verwendet. Die Auflösung der Nanokapseln kann dann beispielsweise durch Entladung oder den Übergang zum jeweils anderen Ladungszustand des Ampholyts erreicht werden.

Die Voraussage des bei einem bestimmten pH-Wert zu erwartenden Ladungszustandes des Gesamtmoleküls z und die Lage des isoelektrische Punktes kann mit folgender Gleichung [1] berechnet werden:

$$z = \sum ni * ((qi-1)+(10^{(pKi-pH)}/(1+10^{(pKi-pH)}))) \qquad [1]$$

Darin ist

- Z die absolute Anzahl der Ladungen an einem Polymermolekül
- ni die Anzahl der funktionellen Gruppen mit einem dazugehörigen pKi und
- qi die absolute Ladung der einzelnen ionischen Gruppe unterhalb ihres pK (Bsp. Carboxyl =0, einfache stickstoffbase = 1, Phosphatgruppe der zweiten Dissoziationsstufe = -1 etc.)

Für Proteine ist dieser Wert in vielen Fällen tabellarisch erfasst oder aus Datenbanken, etwa der SWISS-Prot, zu ermitteln. Die Polyampholyte können je nach ihrem Ladungszustand wie die entsprechenden Polyelektrolyte verwendet werden.

Die erfindungemäßen Nanokapseln werden z.B. hergestellt, indem

i) ein kolloidales Templat in wäßriger Lösung mit einem bestimmten pH-Wert vorgelegt und
ii) komplementär geladene Polymere auf dem Templat abgeschieden werden, wobei mindestens eines der Polymere ein Polyampholyt ist.

Die Nanokapseln können nach ihrer Herstellung aufgelöst werden, indem sich eine pH-Wert-Änderung so vollzieht, dass eine Entladung oder Umladung des Polyampholyten erfolgt. Die Herstellung und Lagerung der Nanokapseln erfolgt also bei einem bestimmten pH-Wert und die Auflösung bei einem veränderten pH-Wert, wobei der isoelektrische Punkt des Polyampholyten zwischen den beiden pH-Werten liegt.

Bei der Herstellung der Kapseln werden jeweils komplementär geladene Polymerschichten, wobei mindestens ein Polymer ein Polyampholyt ist, bei einem bestimmten pH-Wert auf das Templat aufgebracht, so dass bevorzugt jeweils eine beliebige Anzahl - aber mindestens zwei - direkt aufeinanderfolgende unterschiedlich geladene Schichten auf dem Templat vorliegen. Es ist selbstverständlich auch möglich, dass eine Struktur eines Coazervates vorliegt. Durch eine Änderung des pH-Wertes kommt es zu einer Um- oder Entladung des Polyampholyten, so dass zum Teil nicht mehr komplementär geladene Polymerschichten vorliegen. Beispielsweise ist es durch die pH-Wert Änderung möglich, dass gleich geladene Schichten vorliegen. Diese gleich, geladenen Schichten stoßen sich einander ab, was zu einem Auseinanderbrechen der Polymerschichten und folgend zu einer Auflösung der Nano- oder Mikrokapseln führt.

Dieser Prozess ist im Beispiel 1 näher ausgeführt. Hier werden liposomale Nanokapseln unter Verwendung von Serumalbumin hergestellt. Bei dem benutzten pH-Wert von 4 liegt das Albumin als Polykation vor und kann mit dem anionischen Heparin wechselwirken. Es lassen sich stabile Kapseln mit mehreren Schichten der Polymere aufbauen. Wird der pH-Wert des Mediums jedoch auf 7 erhöht, so zerfallen diese Kapseln sehr schnell. Dieser Mechanismus erlaubt eine Reihe von Anwendungen.

Geeignete kolloidale Template nach i) sind anorganische oder organische Mikro- und Nanopartikel, die kolloidal gelöst in wäßrigen Medien vorliegen. Diese Gruppe umfasst insbesondere Mikropartikel einer Größe von weniger als 10 $\mu$m und mehr als 20 nm, bevorzugt weniger 5 $\mu$m und mehr als 50 nm und besonders bevorzugt weniger als 1 $\mu$m und mehr als 70 nm. Besonders geeignete Template sind organische Partikel wie etwa Polymer-Latizes, aber auch Zellen oder Zellbestandteile.

In einer vorteilhaften Ausführungsform sind die Template Kristalle, insbesondere schwer wasserlösliche Kristalle oder Kristalle, deren Oberfläche mit Ladungsträgern modifiziert sein kann. Geeignete Template sind weiterhin Liposomen oder Öl/Wasser-Emulsionen oder Wasser/Öl/Wasser-Doppelemulsionen. Die Herstellung der Nano- oder Mikrokapseln auf diesen Templaten ist dem Fachmann bekannt, die verschiedenen Herstellungsmethoden sind insbesondere für spezifische Verwendung vorteilhaft. Liposomen, o/w-Emulsionen und w/o/w-Emulsionen, aber auch Kristalle als bevorzugte Template zeichnen sich dadurch aus, dass sie sehr leicht, etwa durch Einwirkung von Detergenzien oder Lösungsvermittlern aus den Strukturen zu entfernen sind. Mit Hilfe dieser Template können daher mit Vorteil Hohlkörper im Mikro- oder Nanometermassstab gefertigt werden. Weiterhin kann vorteilhafter weise die Ladung dieser Template sehr leicht durch Variation der Zusammensetzung variiert werden.

Unter Berücksichtigung dieser Gesichtspunkte sind ganz besonders vorteilhaft solche Liposomen und Emulsionen, die einen hohen Anteil geladener Membranbestandteile besitzen. Geeignete Komponenten sind unter anderem geladene amphipatische Verbindungen, die sich in die Grenzschicht einlagern können. Zu den vorteilhaften Verbindungen gehören daher natürliche oder synthetische Phospholipide und deren Derivate, insbesondere Phosphatidylserin, Phosphatidylglycerol oder Phosphatidsäure, aber auch Sphingolipide, Ceramide, Tetraetherlipide oder andere Etherlipide sowie geladene Derivate des Cholesterols wie Cholesterolsulfat, Cholesterolhemisuccinat, Dimethylaminoethylcarbamoyl-Cholesterol und andere dieser Verbindungen. Zu den Verbindungen gehören mit Vorteil weiterhin Alkyl- oder Alkenyl- carbonsäuren, -sulfonsäuren (Laurylsulfat), -amine, - ammoniumsalze (Cetylammoniumbromid), Dialkylamine oder - ammoniumverbindungen wie DOTAP oder DOTIM, Phosphorsäureester mit langkettigen Alkoholen (Cetylphosphat) und weitere membranbildende oder membranständige geladene Verbindungen. Ungeladene Membranbestandteile wie Phosphatidylcholin, Phosphatidylethanolamin, a-Tocopherol, Cholesterol und andere mehr können zusätzlich als membranbildende Komponenten verwendet werden, insbesondere wenn liposomale Template vorliegen.

Vorteilhafte Anteile bei liposomalen Templaten liegen insbesondere zwischen 10 und 50% für sterolartige Ladungsträger und oberhalb von 10% für Ladungsträger auf der Basis von Phospholipiden. Unter den o/w-Emulsionen und den w/o/w-Emulsionen sind solche Formen besonders bevorzugt, die einen hohen Anteil geladener Emulgatoren aufweisen. So liegt vorteilhaft der Anteil geladener Emulgatoren oberhalb 10% der Gesamtemulgatormenge, besonders vorteilhaft oberhalb 30% dieser Menge.

Nach der Bereitstellung des Templats in wäßriger Suspension werden Polyampholyte und/oder Polyelektrolyte

auf den Templaten abgeschieden. Diese Abscheidung erfolgt bevorzugt schichtweise geordnet, so dass komplementär geladene Schichten unmittelbar aufeinander folgen. Eine beliebige Anzahl, mindestens aber zwei dieser Schichten können auf der Oberfläche der Template abgeschieden werden. Ein erfindungswesentliche Merkmal ist nach ii) der Einbau von einem oder mehreren Polyampholyten in solche Nanokapseln. Polyampholyte sind solche wasserlöslichen Polymere, die sowohl saure als auch basische Gruppen enthalten. Je nach pH-Wert des Mediums und Art und Anzahl der ionogenen Gruppen können diese Moleküle als Polyanion, als nach aussen ungeladene Spezies oder als Polykation vorliegen. Polyampholyte haben im Unterschied zu Polysäuren oder Polybasen einen isoelektrischen Punkt, bei dem ihre Nettoladung Null ist.

Wasserlösliche Polyampholyte und Verfahren zu ihrer Herstellung sind in den folgenden Offenbarungen beschrieben; US 3,929,743: synthetische lineare (Co-)Polymere umfassend Carboxylgruppen, die nach der Polymerisation teilweise iminiert werden; US 4,522,708 und WO 034581A1: synthetische lineare Copolymere aus einer Acrylsäure, Acrylamid und Dimethyl- oder Diethyldiallyl-ammoniumhalogenid; US 4,959,163: Synthetische lineare Copolymere aus einfach geladenen oder ungeladenen monoolefinischen Monomeren und einem zwitterionischen monoolefinischen Monomer; US 5,132,285: graft-Copolymere aus Stärke und zwitterionischen Monomeren oder kationischen/anionischen Monomerpaaren; JP 8157501: N-Carboxyacylchitosan durch Umsetzung von Chitosan und Bernsteinsäureanhydrid oder Pthalsäureanhydrid; US 3,673,171: modifizierte amphotere Stärke mit basischen und sauren Gruppen und WO 00/39176, die die Herstellung von hydrophilen Polyampholyten, umfassend eine große Zahl möglicher anionischer oder kationischer Monomere, beschreibt, wobei die kationischen Monomere bevorzugt im Überschuss eingesetzt werden.

In einer bevorzugten Ausführungsform der Erfindung umfassen die Kapseln als Polyampholyte mindestens ein Proteine.

In einer besonders bevorzugten Ausführungsform umfassen die Proteine Albumine, Hämoglobine, Myoglobine, Antikörper, Proteasen, Proteaseinhibitoren, Lipasen, Esterasen, Amylasen, Dehydrogenasen Kollagene, Fibrinogene, Protein A, Protein G, Lektine und/oder Histone. Weitere geeignete Verbindungen sind Peptide, Heteropolymere aus Aminosäuren, umfassend eine Vielzahl von Histidinen, Asparaginsäuren oder Glutaminsäuren, oder acylierte Protamine.

Polyampholyte lassen sich aber auch durch nachträgliche Modifikation bestehender Polymere herstellen, etwa indem ein Dextransulfat oder eine Alginsäure partiell aminiert werden oder indem ein Chitosan oder Polyallylamin partiell carboxyliert, sulfatiert oder phosphoryliert wird.

Polyampholyte können unabhängig von ihrer chemischen Natur nach ihrer Ladungscharakteristik bei verschiedenen pH-Werten eingeteilt werden. Diese Ladungsfunktion wird durch die Gleichung [1] beschrieben.

In einer bevorzugten Ausführungsform der Erfindung weisen die Kapseln mindestens ein Polyampholyt einem isoelektrischen Punkt zwischen 4 und 9 auf. Bevorzugte Polyampholyte sind solche mit einem isoelektrischen Punkt zwischen 4 und 9. Sie umfassen in jedem Fall mindestens eine Klasse funktioneller Gruppen, deren pK zwischen 4 und 9 liegt. Bevorzugte anionische Gruppen sind Carboxylgruppen. Ihrer chemischen Natur nach sind die bevorzugten Kationen Stickstoffbasen wie beispielsweise Piperazine, Imidazole und Morpholine oder Thiomorpholine, Purine oder Pyrimidine sowie Amine an aromatischen Systemen. Bevorzugt- sind solche Molekülfragmente, wie sie in biologischen Systemen vorkommen, also beispielsweise 4-Imidazole (Histamin), 2-, 6- oder 9- Purine (Adenine,Guanine, Adenosine oder Guanosine), 1-, 2- oder 4-Pyrimidine (Uracile, Thymine, Cytosine, uridine, Thymidine, Cytidine) oder auch Pyridin-3-carbonsäuren (Nicotinsäureester oder -amide).

Stickstoffbasen mit bevorzugten pKa-Werten entstehen auch durch einfache oder mehrfache Substitution des Stickstoffatoms mit Niederalkanhydroxylen, etwa Hydroxymethyl- oder Hydroxyethylgruppen. Geeignete organische Basen aus dieser Gruppe sind beispielsweise Aminopropandiole, Triethanolamine, Tris-(hydroxymethyl)methylamine, Bis-(hydroxymethyl)methylamine, Tris-(hydroxyethyl)methylamine, Bis-(hydroxyethyl)methylamine oder die entsprechend substituierten Ethylamine.

Besonders bevorzugte Polyampholyte enthalten sowohl anionische als auch kationische Ladungsträger aus den oben genannten Gruppen. Weiter bevorzugt sind Polyampholyte, bei denen die pK-Werte der Ladungsträger nicht mehr als 3 Einheiten auseinander liegen. Ganz besonders bevorzugt sind Polyampholyte, bei denen die pK-Werte der Ladungsträger nicht mehr als 2 Einheiten auseinander liegen.

Zusätzlich können weitere Funktionen auf solchen Polymeren vorhanden sein, so etwa ionische Gruppen mit einem pK-Wert außerhalb des genannten Bereiches oder nichtionische polare Gruppen wie Hydroxylfunktionen oder Amidgruppen zur Erhöhung der Wasserlöslichkeit. Die oben beschriebenen funktionellen Gruppen sind jedoch notwendig für die Funktion des Ampholyten im bevorzugten pH-Bereich.

In einer weiteren bevorzugten Ausführungsform umfassen die Polyampholyte ethylenisch ungesättigten Struktureinheiten. Polyampholyte können als statistische lineare Copolymere vorliegen, bei denen anionische und kationische Gruppen einander unregelmässig abwechseln. Mit Vorteil können für die Ausführung der Erfindung weitere spezialisierte Polymere verwendet werden, die eine Vielzahl identischer zwitterionischer Struktureinheiten umfassen.

In einer besonders bevorzugten Ausführungsform der Erfindung umfassen die Polyampholyte zwitterionische Struktureinheiten aus acrylsubstituierten Hisitidinen, Diaminobenzoesäuren, Imidazoldicarbonsäuren; Maleinsäuremonoimidazolen,- morpholinen, -thiomorpholinen; der Itaconsäuremonoimidazole, - morpholine, -thiomorpholine; der Aconitsäuremonoimidazole, - morpholine,-thiomorpholine und/oder -piperazine. Die folgende Tabelle gibt besonders bevorzugte Ausführungen für Struktureinheiten aus ethylenisch ungesättigten Monomeren wieder:

| | |
|---|---|
| 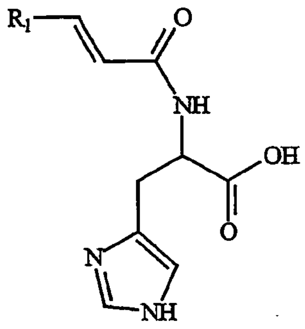 | Acrylamido-Histidine. Der Rest R1 kann Wasserstoff, Methyl; Ethyl, Propyl, Isopropyl, Hydroxymethyl, Hydroxyethyl, Hydroxypropyl oder Hydroxyisopropyl sein. Anstelle der Acrylsäure kann sinngemäss auch Crotonsäure stehen. |
| 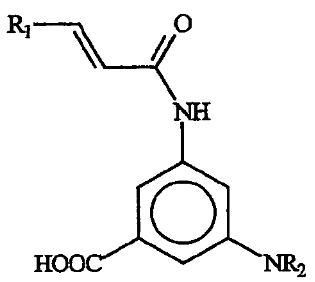 | N-(Acrylamido)-Diaminobenzoesäuren. Für den Rest R1 und die ethylenisch ungesättigte Gruppe gelten die oben gemachten Ausführungen. R2 ist unabhängig davon Wasserstoff, Methyl,. Ethyl, Propyl, Isopropyl, Hydroxymethyl, Hydroxyethyl, Hydroxypropyl oder Hydroxyisopropyl oder eine Kombination dieser Reste. |
| 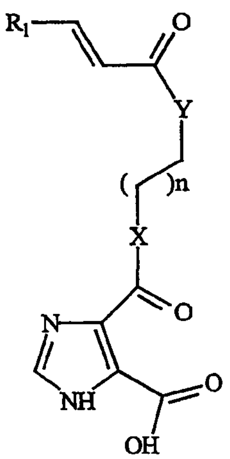 | Monomere umfassend Acrylsäure und Imidazol-(4,5)-dicarbonsäure. Der Rest R1 kann Wasserstoff, Methyl, Ethyl, Propyl, Isopropyl, Hydroxymethyl, Hydroxyethyl, Hydroxypropyl oder Hydroxyisopropyl sein. Anstelle der Acrylsäure kann sinngemäss auch Crotonsäure stehen.<br>Die beiden funktionellen Elemente sind über einen Spacer miteinander verbunden. X und Y sind bevorzugt unabhängig voneinander Stickstoff oder Sauerstoff, die zwischen den beiden Heteroatomen liegende Alkylkette hat 0 bis 8 C-Atome und ist linear oder verzweigt oder zyklisch geschlossen und kann weitere Hydroxylfunktionen enthalten. Der Spacer kann auch ein Polyethylenglykol sein. |

(fortgesetzt)

| | |
|---|---|
| 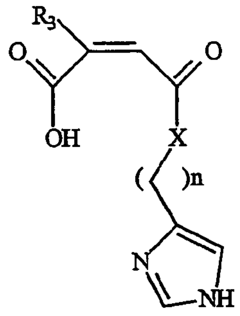 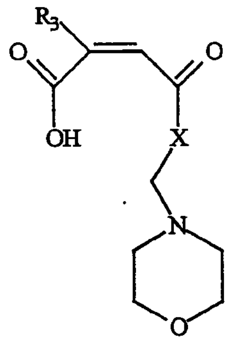 | Maleinsäuremonoimidazole oder -morpholine oder -thiomorpholine. Der Rest R3 kann Wasserstoff, Methyl, Ethyl, Propyl, Isopropyl, Hydroxymethyl, Hydroxyethyl, Hydroxypropyl oder Hydroxyisopropyl sein. Der Rest R3 kann auch eine weitere Carbonsäurefunktion umfassen, insbesondere kann anstelle der Maleisäure Aconitsäure stehen.<br>X ist Sauerstoff oder Stickstoff, die zwischen X und dem kationischen Rest liegende Alkylkette hat 0 bis 8 C-Atome und ist linear oder verzweigt oder zyklisch geschlossen und kann weitere Hydroxylfunktionen enthalten. ( )n kann auch ein Polyethylenglykol sein. |
| 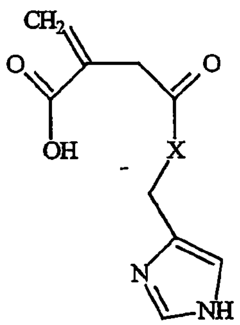 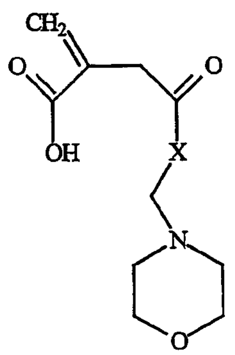 | Itaconsäuremonoimidazole oder -morpholine oder -thiomorpholine.<br>X ist Sauerstoff oder Stickstoff, die zwischen X und dem kationischen Rest liegende Alkylkette hat 0 bis 8 C-Atome und ist linear oder verzweigt oder zyklisch geschlossen und kann weitere Hydroxylfunktionen enthalten. ( )n kann auch ein Polyethylenglykol sein. |

(fortgesetzt)

| | |
|---|---|
| 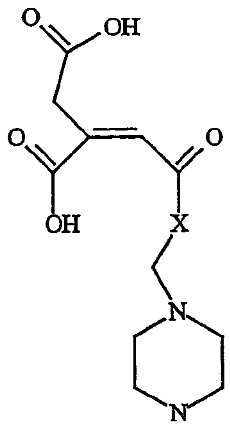 | Aconitsäuremonopiperazine.<br>X ist Sauerstoff oder Stickstoff, die zwischen X und dem Piperazin liegende Alkylkette hat 0 bis 8 C-Atome und ist linear oder verzweigt oder zyklisch geschlossen und kann weitere Hydroxylfunktionen enthalten. ( )n kann auch ein Polyethylenglykol sein.<br>Das Piperazin kann an der N'-Position weiter substituiert sein, insbesondere mit einem Methyl, Ethyl, Propyl, Isopropyl, Hydroxymethyl, Hydroxyethyl, Hydroxypropyl oder Hydroxy-isopropylrest. |
| 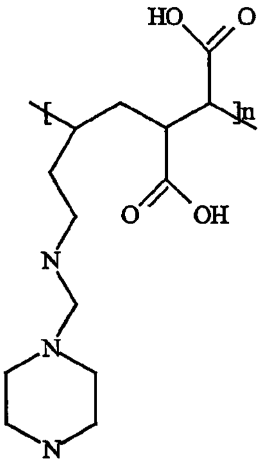 | Additionsprodukt aus Aminoethylpiperazin und Poly-(alt-Maleinsäure/ Butadien). Anstelle des Piperazins können auch Morpholin, Thiomorpholin oder Imidazol stehen. |

Polymere, umfassend die genannten Struktureinheiten können sowohl durch Polymerisation der hier gezeigten Monomere entstehen, aber auch als graft-Polymere aus Vorläufern. Geeignete Precursoren sind etwa Poly-(alt-Maleinsäure- anhydrid/Ethylen) oder auch Poly-(acrylsäureanhydrid) oder Poly-(acryloylchlorid), aus denen leicht die entsprechenden Ester oder Amide zugänglich sind.

Die Polyampholyte können weitere, insbesondere nichtionische Monomere enthalten. Besonders bevorzugte Ausführungsformen sind:

- statistische oder alternierende Copolymerisate der Acrylate oder Crotonate mit Acrylamid.
- alternierende Copolymerisate der Maleate, Itaconate oder Aconitate mit Ethylen oder Butadien.

Die Polymere aus ethylenisch ungesättigten Monomeren sind zumindest in ihrem Rückgrat nicht biologisch abbaubar. Sollen diese Polymere bei pharmazeutischen Anwendungen in den Körper gebracht werden, so ist deren Akkumulation nur durch Ausscheidung zu verhindern. Solche Polymere haben bevorzugt eine Molmasse von weniger als 100 kDa, besonders bevorzugt weniger als 30 kDa. Für ihre Funktionaliätt beim Aufbau von Polyelektrolytmultischichten sind Molmassen von mehr als 10 kDa vorteilhaft.

Biologisch abbaubare Polymere sind aus den Polyzuckern zugänglich. Vorteilhaft ist die Modifizierung von mindestens 20% bis zu 80% der Carboxylgruppen eines Alginats durch kationische Komponenten mit einem pK im Bereich zwischen 4 und 9. Geeignete Ladungsträger sind oben genannt.

Die folgende Tabelle gibt vorteilhafte Ausführungen für bevorzugte Strukturelemente in Polyzuckern.

| | |
|---|---|
| 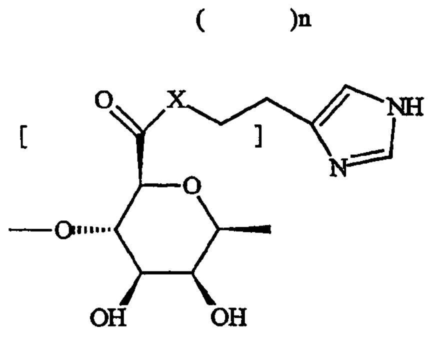 | Amide oder Ester der Alginsäure. X bedeutet Stickstoff oder Sauerstoff,<br>( )n ist eine Alkylkette mit 0 bis 8 C-Atomen und ist linear oder verzweigt oder zyklisch geschlossen und kann weitere Hydroxylfunktionen enthalten. ( ) n kann auch ein Polyethylenglykol sein. Als kationische Gruppe kann insbesondere auch Morpholin, Thiomorpholin, Diaminobenzoesäure oder Piperazin stehen. |
| 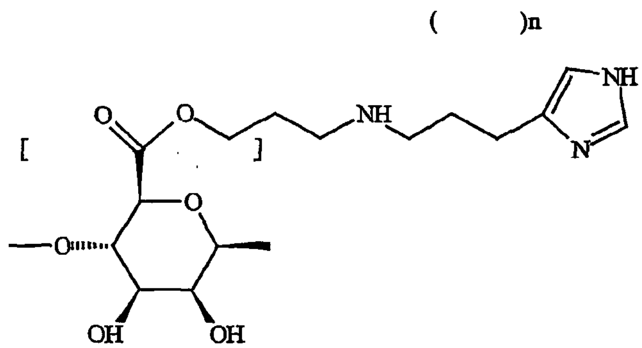 | Aminoester der Alginsäure.<br>( )n ist eine Alkylkette mit 0 bis 8 C-Atomen und ist linear oder verzweigt oder zyklisch geschlossen und kann weitere Hydroxylfunktionen enthalten.<br>( )n kann auch ein Polyethylenglykol sein. Als kationische Gruppe kann insbesondere auch Morpholin, Thiomorpholin, Diaminobenzoesäure oder Piperazin stehen. |
| 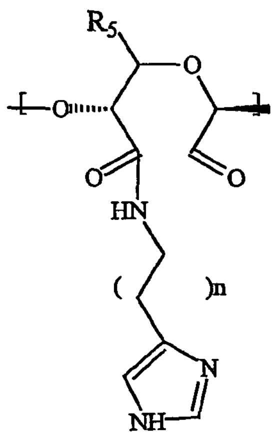 | reduzierte Schiffbasen aus Aminoimidazol und oxidierten Dextranen. R5 kann Wasserstoff, Hydroxymethyl oder eine Carbonsäure sein.<br>( )n ist eine Alkylkette mit 0 bis 8 C-Atomen und ist linear oder verzweigt oder zyklisch geschlossen und kann weitere Hydroxylfunktionen enthalten.<br>( )n kann auch ein Polyethylenglykol sein. Als kationische Gruppe kann insbesondere auch Morpholin, Thiomorpholin, Diaminobenzoesäure oder Piperazin stehen. |
| 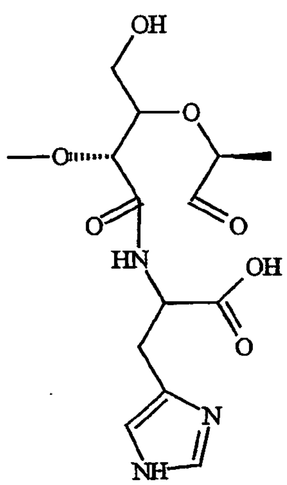 | reduzierte Schiffbase aus oxdiertem Dextran und Histidin. Anstelle des linearen Dextrans kann auch Stärke oder Glykogen stehen. Anstelle des Histidins kann auch Diaminobenzoesäure stehen. |

Die Erfindung lässt sich mit Vorteil auch durch den Einsatz von carboxylierten Polyethyleniminen realisieren. Diese Derivate sind durch Umsetzung der Polyethylenimine mit Halogencarbonsäuren, beipsielsweise Chloressigsäure, ethylenisch ungesättigten Carbonsäuren, beipsielsweise Acrylsäure, Methacrylsäure, Crotonsäure, Maleinsäure, Itconsäure, Aconitsäure, oder mit Säurechloriden oder - anhydriden zugänglich beispielsweise Maleinsäureanhydrid, Itaconsäureanhydrid, Aconitsäureanhydrid, Ethylendiaminoessigsäuredianhydrid.

In einer weiteren bevorzugten Ausführungsform umfassssen die Polyampholyte modifizierte Alginsäuren, Dextrane, Stärken, Glykogenen, Polyglycerolen, Guar Gummis oder Pektine sind, wobei diese mindestens einen Substituenten der Imidazole, Morpholine, Thiomorpholine, Piperazine, Histidine und/oder Diaminobenzoesäuren.

Die Erfindung betrifft auch die Verwendung der Nano- oder Mikrokapseln zur Verpackung und Freisetzung von Wirkstoffen.

In einer bevorzugten Ausführungsform werden die Kapseln zur oralen Wirkstoffapplikation verwendet, wobei eine Polymerhülle einen solchen pH-Wert aufweist, dass die Kapseln bei einem pH-Wert >=7 zerfallen. Dem Fachmann ist bekannt, welchen isoelektrischen Punkt der Polyampholyt hierzu aufweisen muss und wie dieser eingestellt werden kann. In einer vorteilhaften Verwendung solcher Nanokapseln werden diese für ein orales Transfersystem verwendet. Dabei erfolgt die Herstellung und Lagerung des wirkstofftragenden Systems bei dem oben beschriebenen leicht sauren pH-Wert. Lösungen mit diesem pH sind problemlos oral aufnehmbar und passieren den sauren Mageninhalt in einer stabilen Form. Mit dem Erreichen des Zwölffingerdarms erfolgt eine Neutralisierung der Lösung, damit zerfallen die Hüllschichten und geben das Templat frei.

Besonders bevorzugt kann dieses System auf Basis von liposomalen Templaten verwendet werden. Die Liposomen können vom Fachmann so ausgeführt werden, dass sie dicht gegenüber pH-Schwankungen des Aussenmediums sind und dadurch den Wirkstoff vor dem Angriff der Magensäure schützen. Solche Liposomen sind beispielsweise für das remote-loading von Wirkstoffen entwickelt worden und im Stand der Technik bekannt. Nach dem Abwerfen der äußeren Hüllschicht stellt die Lipidmembran ihrerseits eine letzte Barriere für die Freisetzung eines eingeschlossenen Wirkstoffs dar. Eine letztliche Freisetzung aus den Liposomen ist aber durch die Aktivität der im Darm vorhandenen Phospholipasen und durch den Gallensaft gegeben.

In einer weiteren bevorzugten Ausführungsform werden die Kapseln zum Transport von Wirkstoffen in Zellen und/oder in Tumorgewebe verwendet, wobei die Kapseln bei einem pH-Wert <=7 zerfallen.

Eine weitere Möglichkeit des Transports von Wirkstoffen besteht in der Nutzung relativ fusogener Liposomen, die ihren Wirkstoff direkt in Zellen der Mucosa abgeben. Solche Liposomen sind dem Fachmann bekannt und enthalten größere Anteile von Phosphatidylethanolamin und/oder kationische Lipide. Diese Liposomen können mit Hüllen, umfassend Polyampholyte, bis in den Darm gebracht werden und geben dann den Wirkstoff direkt, in die Zellen der Darmwand ab. In diesem Fall wird jeglicher Kontakt des Wirkstoffes mit dem Verdauungsapparat vermieden.

In einer weiteren vorteilhaften Verwendung der Erfindung werden die schaltbaren Nanokapseln zur rektalen Applikation von Wirkstoffen eingesetzt. Hier können die eigentlichen Wirkstoffträger wie Liposomen oder Emulsionen in rektal gut applizierbaren Darreichungsformen wie beispielsweise Suppositorien bei der Herstellung und Lagerung stabilisiert werden.

Eine weitere vorteilhafte Anwendung der erfinderischen Lehre lässt sich mit der gegensätzlich geladenen Kombination aus anionischem Protein - Albumin oder Kollagen bei pH >6, Hämoglobin bei pH>7.5 - und einem Polykation wie beispielsweise Chitosan, Polylysin oder Polyallylamin herstellen. Solche Nanokapseln sind sensitiv gegenüber sauren Milieus und können bei der Freisetzung von Wirkstoffen während der Endozytose, im Innern von Tumoren oder im Hautschweiss verwendet werden.

In einer besonders bevorzugten Ausführungsform werden die Kapseln zur Freisetzung von Wirkstoffen auf der Haut und/oder für kosmetische Zwecke, wobei die Kapseln bei einem pH-Wert <=7 zerfallen.

Eine weitere vorteilhafte Verwendung betrifft die Stabilisierung von Liposomen oder Emulsionen oder Doppelemulsionen in kosmetischen und pharmazeutischen Cremes oder Salben. Insbesondere Liposomen oder Doppelemulsionen sind in den gängigen Formulierungen instabil und geben den eingeschlossenen Wirkstoff schon während der Lagerung des Produktes frei. Eine Nanoverkapselung dieser Strukturen kann unter Verwendung von Polyampholyten so ausgeführt werden, dass die eigentlichen Wirkstoffträger während der Lagerung stabilisiert werden, aber auf der Haut wieder entkapselt vorliegen.

In einer weiteren bevorzugten Ausführungsform werden die Kapseln zur Verkapselung und Freisetzung von Waschmittelenzymen verwendet, wobei die Kapseln bei einem pH-Wert <=9 zerfallen. Konzentrierte Flüssigwaschmittel haben einen pH-Wert zwischen 9 und 11, der beim Verdünnen in die Waschlösung auf Werte um 8 sinkt. Unter diesen Bedingungen verringert sich die Ladung der verwendeten Proteasen erheblich und die Kapseln lösen sich mit der Verdünnung vollständig auf.

Besonders bevorzugt ist es, Waschmittelenzyme zu verwenden, die Proteasen und/oder Subtilisin umfassen. In dieser vorteilhaften Anwendungsform werden Nanokapseln aus Subtilisin und einem Polykation wie etwa Polyallylamin, Polyquaternium, Polyvinylpyridin, kationischer Stärke oder ähnlichen Verbindungen hergestellt. Die Protease als Wirkstoff ist in diesem Fall Bestandteil der Hüllschicht, als Templat werden entfernbare und ökologisch unbedenkliche

Strukturen wie Liposomen oder Emulsionen eingesetzt. Durch die sehr geringe Größe der Strukturen tritt optisch keine Trübung des Produkts auf. Ein weiterer Vorteil ist durch den gleichzeitig möglichen Einsatz von anderen Waschmittelenzymen, wie etwa Zellulasen oder Lipasen gegeben. Solche Kombinationen sind mit konventionellen Formulierungen nicht herstellbar, da freie Protease zum schnellen Abbau der anderen Enzyme führt.

Die erfindungsgemäßen Nanokapseln weisen gegenüber den bekannten Nanokapseln mehrere Vorteile auf. Bekannte Nanokapseln bestehen bisher unabhängig von ihrem Templat aus wasserlöslichen Polymeren, die durch ionische Wechselwirkungen zusammengehalten werden. Wesentlich für ihre Struktur ist das Vorhandensein einer Vielzahl von wechselwirkenden Gruppen an den verwendeten Polymeren. Durch die beim Aufbau stattfindende Vernetzung wird eine hohe Stabilität der Gesamtstruktur erzeugt, was zu einer extrem hohe Stabilität der Nanokapseln führt und die Freisetzung der eingeschlossenen Substanzen verhindert. Ein besonderer Vorteil der Verwendung von Polyampholyten für den Aufbau von Nanokapseln besteht darin, dass diese Kapseln unter einfachen Bedingungen wieder aufgelöst werden können. Dadurch sind diese Nanokapseln, insbesondere deren Hüllstruktur, als ein vielseitig nutzbares Verpakkungs- und Freisetzungssystem nutzbar.

Weitere vorteilhafte Ausgestaltungsformen ergeben sich aus der Beschreibung.

Im folgenden soll die Erfindung anhand von Ausführungsbeispielen veranschaulicht werden, ohne die Erfindung darauf einzuschränken.

**Beispiel 1**

Liposomale Nanokapseln aus Albumin und Heparin

Herstellung der Liposomen 20 mol-% Dipalmitoylphosphatidylcholin und 80 mol-% Dipalmitoyl-phosphatidylglycerol werden in Isopropanol gelöst und unter Vakuum bis zur Trockene eingedampft. Der Lipidfilm wird anschließend in soviel Puffer (10mM Hepes, 150mM NaCl pH7,5) rehydratisiert, dass eine Lipidkonzentration von 25mM erreicht wird. Die Suspension wird mindestens einmal eingefroren, bei 50˚C wieder aufgetaut und dann mehrmals durch isopore Polykarbonatmembranen mit einer Porengröße von 200nm gedrückt.

Beschichtung mit Albumin und Heparin und Analyse der Strukturen Die Polymere werden in den Konzentrationen von 1mg/ml und 5mg/ml in Puffer (10mM Natriumacetat, pH4) gelöst. Die Liposomen werden in Puffer (10mM Natriumacetat, pH4) auf eine Lipidkonzentration von 0,2mM verdünnt. Zu 50ml dieser verdünnten Liposomen werden nacheinander geeignete Mengen der beiden Polymere (siehe Tabelle) zugemischt. Die jeweils eingesetzte Menge Polymer bindet hinreichend vollständig an die Partikeloberfläche, so dass keine Reinigungsschritte zwischengeschaltet werden müssen.

| Schicht (S) | mg Polymer/mg Lipid |
|---|---|
| S1 BSA | 1,00 |
| S2 Heparin | 0,33 |
| S3 BSA | 4,75 |
| S4 Heparin | 1,59 |
| S5 BSA | 12,66 |
| S6 Heparin | 4,22 |

100μl der Suspension werden anschließend durch Zugabe von 100μl HEPES-Puffer (200mM, pH7.5) neutralisiert, die Probe wird dann durch ein Filter der Weite 0,0μm filtriert. Die enzymatische Aktivität kann im Filtrat nachgewiesen werden. Das ist nicht der Fall, wenn die zu filtrierende Suspension einen pH-Wert von weniger als 5 aufweist.

**Beispiel 2**

Liposomale Nanokapseln aus Subtilisin und Polyallylamin

Liposomen mit einer Zusammensetzung aus 40 Mol-% Cholesterylhemisuccinat und 60 Mol-% Soja-Lecithin werden wie oben durch Extrusion hergestellt. Subtilisin wird mit einer Konzentration von 5mg/ml und Polyallyamin mit einer Konzentration von 1mg/ml in Puffer (100mM Natriumcarbonat, pH 10.0) gelöst. Die Liposomen werden in Wasser auf eine Konzentration von 0.2mM verdünnt. Die unten aufgeführten Mengen Polymer werden nacheinander unter schnellem Rühren zu den Liposomen gegeben:

S1 Polyallyamin 80μg/mg Lipid

(fortgesetzt)

| | |
|---|---|
| S2 Subtilisin | 220μg/mg Lipid |
| S3 Polyallylamin | 150μg/mg Lipid |
| S4 Subtilisin | 840μg/mg Lipid |
| S5 Polyallyamin | 230μg/mg Lipid. |

Die schichtweise Abscheidung des Polymers kann durch Messungen des Zetapotentials verfolgt werden.

100μl der Suspension werden anschließend durch Zugabe von 100μl Phosphatpuffer (100mM, pH6.5) neutralisiert, die Probe wird dann durch ein Filter der Weite 0,1μm filtriert. Die enzymatische Aktivität kann im Filtrat nachgewiesen werden. Das ist nicht der Fall, wenn die zu filtrierende Suspension einen pH-Wert von mehr als 9 aufweist.

**Beispiel 3**

Synthese eines Polyampholyten 1

5g Poly-(alt-ethylene/maleic anhydride), 100kDa, werden in 100ml trockenem Dioxan gelöst. Dazu wird langsam 50ml einer Lösung von 60mMol 2-Aminoethylmorpholin in Dioxan gegeben. Die Lösung wird 2 Stunden bei 50˚C gerührt und am Rotationsverdampfer auf 50ml eingeengt. Dem Produkt werden 500ml Wasser zugesetzt und der pH-Wert wird auf 7 eingestellt. Dioxan und überschüssiges Aminoethylmorpholin werden durch Ultrafiltration entfernt, das Polymer wird auf 100ml eingeengt und anschließend lyophilisiert.

**Beispiel 4**

Synthese eines Polyampholyten 2

2g Dextran (70kDa) werden in 100ml Puffer (10mM HEPES, 150mM NaCl pH 8,0) gelöst. Zur Lösung werden unter Rühren nacheinander 50mMol L-Histidin und 20mMol Natriumperiodat gegeben, es wird 5 Stunden bei 60˚C gerührt. Dabei wird der pH-Wert in einem Bereich zwischen 7 und 8 gehalten.
Nach dem Abkühlen werden 50mMol Natriumborhydrid zugegeben , die Mischung wird dann über Nacht weiter gerührt. Die Reinigung des Polymers erfolgt wie oben durch Ultrafiltration, zur Lagerung wird das Produkt lyophilisiert.

**Patentansprüche**

**1.** Auflösbare Nano- oder Mikrokapseln, umfassend ein kolloidales Templat in wässriger Lösung und komplementär geladene Polymere auf einem Templat, wobei mindestens eines der Polymere ein Polyampholyt ist, welcher durch eine pH-Wertänderung ent- oder umladbar ist und wobei jedes Templat individuell von einer Hüllstruktur aus geladenen Polymeren umgeben ist.

**2.** Kapseln nach Anspruch 1,
**dadurch gekennzeichnet, dass**
das Templat ein Liposom, eine Öl- in Wasser- Emulsion, eine Wasser/Öl/Wasser-Doppelemulsion, einen anorganischen und/oder organischen Kristall umfasst.

**3.** Kapseln nach Anspruch 1 oder 2,
**dadurch gekennzeichnet, dass**
die Differenz der pK-Werte von mindestens zwei Ladungsträgern der Polyampholyte nicht mehr als 3 Einheiten umfasst, besonders bevorzugt nicht mehr als 2 Einheiten.

**4.** Kapseln nach einem der Ansprüche 1 bis 3,
**dadurch gekennzeichnet, dass**
mindestens ein Polyampholyt einen isoelektrischen Punkt zwischen 4 und 9 aufweist.

**5.** Kapseln nach einem der Ansprüche 1 bis 4,
**dadurch gekennzeichnet**, das
die Polyampholyte Proteine umfassen.

6. Kapseln nach einem der Ansprüche 1 bis 5,
**dadurch gekennzeichnet, dass**
die Proteine Albumine, Hämoglobine, Myoglobine, Antikörper, Proteasen, Proteaseinhibitoren, Lipasen, Esterasen, Amylasen, Dehydrogenasen Kollagene, Fibrinogene, Protein A, Protein G, Lektine und/oder Histone umfassen.

7. Kapseln nach einem der Ansprüche 1 bis 6,
**dadurch gekennzeichnet, dass**
die Polyampholyte ethylenisch ungesättigten Struktureinheiten umfassen.

8. Kapseln nach einem der Ansprüche 1 bis 7,
**dadurch gekennzeichnet, dass**
die Polyampholyte zwitterionische Struktureinheiten aus acrylsubstituierten Hisitidinen, Diaminobenzoesäuren, Imidazoldicarbonsäuren; Maleinsäuremonoimidazolen,- morpholinen, -thiomorpholinen; der Itaconsäuremonoimidazole, -morpholine, -thiomorpholine; der Aconitsäuremonoimidazole, -morpholine,-thiomorpholine oder -piperazine umfassen.

9. Kapseln nach einem der Ansprüche 1 bis 8,
**dadurch gekennzeichnet, dass**
die Polyampholyte modifizierte Alginsäuren, Dextrane, Stärken, Glykogenen, Polyglycerolen, Guar Gummis oder Pektine sind, wobei diese mindestens einen Substituenten der Imidazole, Morpholine, Thiomorpholine, Piperazine, Histidine und/oder Diaminobenzoesäuren umfassen.

10. Verfahren zur Herstellung von Nano- oder Mikrokapseln nach einem der Ansprüche 1 bis 9 in dem ein kolloidales Templat in wäßriger Lösung von Polymeren umhüllt wird,
**dadurch gekennzeichnet, dass**
die Polymere komplementär geladen und mindestens ein Polymer ein Polyampholyt ist.

11. Verwendung der Nano- oder Mikrokapseln nach einem der Ansprüche 1 bis 9 zur Verpackung und Freisetzung von Wirkstoffen.

12. Verwendung der Kapseln nach Anspruch 11 zur oralen Wirkstoffapplikation,
**dadurch gekennzeichnet, dass**
die Kapseln bei einem pH-Wert >=7 zerfallen.

13. Verwendung der Kapseln nach Anspruch 11 zum Transport von Wirkstoffen in Zellen und/oder in Tumorgewebe,
**dadurch gekennzeichnet, dass**
die Kapseln bei einem pH-Wert <=7 zerfallen.

14. Verwendung der Kapseln nach Anspruch 11 zur Freisetzung von Wirkstoffen auf der Haut und/oder für kosmetische Zwecke,
**dadurch gekennzeichnet, dass**
die Kapseln bei einem pH-Wert <=7 zerfallen.

15. Verwendung der Kapseln nach Anspruch 11 zur Verkapselung und Freisetzung von Waschmittelenzymen,
**dadurch gekennzeichnet, dass**
die Kapseln bei einem pH-Wert <=9 zerfallen.

16. Verwendung der Kapseln nach Anspruch 14,
**dadurch gekennzeichnet**, das
die Waschmittelenzyme Proteasen und/oder Subtilisin umfassen.

## Claims

1. Dissolvable nano- or microcapsules comprising a colloidal template in aqueous solution and polymers of complementary charge on a template, at least one of said polymers being a polyampholyte which can be discharged or recharged by altering the pH value, and each template being individually surrounded by an envelope structure of charged polymers.

2. The capsules according to claim 1,
**characterized in that**
the template comprises a liposome, an oil-in-water emulsion, a water/oil/water double emulsion, an inorganic and/or organic crystal.

3. The capsules according to claim 1 or 2,
**characterized in that**
the difference of the pK values of at least two charge carriers of the polyampholytes comprises no more than 3 units and especially preferably no more than 2 units.

4. The capsules according to any of claims 1 to 3,
**characterized in that**
at least one polyampholyte has an isoelectric point between 4 and 9.

5. The capsules according to any of claims 1 to 4,
**characterized in that**
the polyampholytes comprise proteins.

6. The capsules according to any of claims 1 to 5,
**characterized in that**
the proteins comprise albumins, hemoglobins, myoglobins, antibodies, proteases, protease inhibitors, lipases, esterases, amylases, dehydrogenases, collagens, fibrinogens, protein A, protein G, lectins and/or histones.

7. The capsules according to any of claims 1 to 6,
**characterized in that**
the polyampholytes comprise ethylenically unsaturated structural units.

8. The capsules according to any of claims 1 to 7,
**characterized in that**
the polyampholytes comprise zwitterionic structural units of acryl-substituted histidines, diaminobenzoic acids, imidazoledicarboxylic acids, maleic acid monoimidazoles, -morpholines, -thiomorpholines; itaconic acid monoimidazoles, -morpholines, -thiomorpholines; aconitic acid monoimidazoles, -morpholines, -thiomorpholines or -piperazines.

9. The capsules according to any of claims 1 to 8,
**characterized in that**
the polyampholytes are modified alginic acids, dextrans, starches, glycogens, polyglycerols, guar gums or pectins, the above comprising at least one substituent of imidazoles, morpholines, thiomorpholines, piperazines, histidines and/or diaminobenzoic acids.

10. A method of producing the nano- or microcapsules according to any of claims 1 to 9, in which method a colloidal template in aqueous solution is enveloped by polymers,
**characterized in that**
the polymers have complementary charge and at least one polymer is a polyampholyte.

11. Use of the nano- or microcapsules according to any of claims 1 to 9 for packaging and releasing active substances.

12. The use of capsules according to claim 11 in oral application of active substances,
**characterized in that**
the capsules disintegrate at a pH value of ≥ 7.

13. The use of capsules according to claim 11 for the transport of active substances in cells and/or tumor tissue,
**characterized in that**
the capsules disintegrate at a pH value of ≤ 7.

14. The use of capsules according to claim 11 for the release of active substances on the skin and/or for cosmetic purposes,
**characterized in that**

the capsules disintegrate at a pH value of ≤ 7.

**15.** The use of capsules according to claim 11 in encapsulation and release of detergent enzymes,
**characterized in that**
the capsules disintegrate at a pH value of ≤ 9.

**16.** The use of capsules according to claim 14,
**characterized in that**
the detergent enzymes comprise proteases and/or subtilisin.

**Revendications**

**1.** Nano- ou microcapsules solubles comprenant une matrice colloïdale en solution aqueuse et des polymères chargés de manière complémentaire sur une matrice, dans lesquelles au moins un des polymères est un polyampholyte, qui peut être déchargé ou dont on peut inverser la charge par modification du pH, et dans lesquelles chaque matrice est entourée individuellement d'une structure enveloppante constituée de polymères chargés.

**2.** Capsules selon la revendication 1,
**caractérisées en ce que**
la matrice est un liposome, une émulsion huile-dans-eau, une émulsion double eau/huile/eau, un cristal minéral et/ou organique.

**3.** Capsules selon la revendication 1 ou 2,
**caractérisées en ce que**
la différence des valeurs de pK d'au moins deux porteurs de charge des polyampholytes n'est pas supérieure à trois unités, de préférence à deux unités.

**4.** Capsules selon l'une des revendications 1 à 3,
**caractérisées en ce**
**qu'**au moins un polyampholyte présente un point isoélectrique entre 4 et 9.

**5.** Capsules selon l'une des revendications 1 à 4,
**caractérisées en ce que**
les polyampholytes comprennent des protéines.

**6.** Capsules selon l'une des revendications 1 à 5,
**caractérisées en ce que**
les protéines comprennent des albumines, des hémoglobines, des myoglobines, des anticorps, des protéases, des inhibiteurs de protéase, des lipases, des estérases, des amylases, des déshydrogénases, des collagènes, des fibrinogènes, de la protéine A, de la protéine G, des lectines et/ou des histones.

**7.** Capsules selon l'une des revendications 1 à 6,
**caractérisées en ce que**
les polyampholytes comprennent des unités structurelles éthyléniquement insaturées.

**8.** Capsules selon l'une des revendications 1 à 7,
**caractérisées en ce que**
les polyampholytes comprennent des unités structurelles zwitterioniques d'histidines acryl-substituées, d'acides diaminobenzoïques, d'acides imidazoledicarboxyliques ; d'acide maléique-monoimidazoles, -morpholines ou -thiomorpholines ; d'acide itaconique-monoimidazoles, -morpholines, -thiomorpholines; d'acide aconitique-monoimidazoles, -morpholines, -thiomorpholines ou -pipérazines.

**9.** Capsules selon l'une des revendications 1 à 8,
**caractérisées en ce que**
les polyampholytes sont des acides alginiques modifiés, des dextranes, des amidons, des glycogènes, des polyglycérols, des gommes de guar ou des pectines, moyennant quoi ceux-ci comprennent au moins un substituant des imidazoles, des morpholines, des thiomorpholines, des pipérazines et/ou des acides diaminobenzoïques.

**10.** Procédé de préparation de nano- ou microcapsules selon l'une des revendications 1 à 9 dans lequel on enrobe une matrice colloïdale en solution aqueuse de polymères,
**caractérisé en ce que**
les polymères sont chargés de manière complémentaire et qu'au moins un polymère est un polyampholyte.

**11.** Utilisation des nano- ou microcapsules selon l'une des revendications 1 à 9 pour empaqueter et libérer des principes actifs.

**12.** Utilisation des capsules selon la revendication 11 pour l'administration orale d'un principe actif,
**caractérisée en ce que**
les capsules se désintègrent à un pH $\geq$ 7.

**13.** Utilisation des capsules selon la revendication 11 pour le transport de principes actifs dans les cellules et/ou les tissus tumoraux,
**caractérisée en ce que**
les capsules se désintègrent à un pH $\leq$ 7.

**14.** Utilisation des capsules selon la revendication 11 pour la libération de principes actifs sur la peau et/ou pour des objectifs cosmétiques,
**caractérisée en ce que**
les capsules se désintègrent à un pH $\leq$ 7.

**15.** Utilisation des capsules selon la revendication 11 pour l'empaquetage et la libération d'enzymes détergentes,
**caractérisée en ce que**
les capsules se désintègrent à un pH $\leq$ 9.

**16.** Utilisation des capsules selon la revendication 14,
**caractérisée en ce que**
les enzymes détergentes comprennent des protéases et/ou de la subtilisine.